# EUROPEAN PATENT APPLICATION

(11) **EP 4 331 663 A1**
(43) Date of publication of application: **06.03.2024**
(21) Application number: 22795657.0
(22) Date of filing: 21.04.2022
(51) Int. Cl.: A61N 5/06

(54) **LIGHT IRRADIATION SYSTEM**

(30) Priority: 27.04.2021 JP 2021075308; 28.06.2021 JP 2021106968
(71) Applicant: Kyocera Corporation, Kyoto-shi Kyoto 612-8501 (JP)
(72) Inventor: KUSHIDA, Nobuko, Kyoto-shi, Kyoto 612-8501 (JP); KYOMOTO, Masayuki, Kyoto-shi, Kyoto 612-8501 (JP); ISHIMIZU, Keita, Kyoto-shi, Kyoto 612-8501 (JP); YAMAGUCHI, Ayaka, Kyoto-shi, Kyoto 612-8501 (JP)
(74) Representative: TBK
(86) International application number: PCT/JP2022/018362
(87) International publication number: WO 2022/230744

(57) **Abstract**

A light irradiation system includes an irradiator, a time measurer, and a controller, and alleviates pain of a subject. The irradiator irradiates a predetermined part of the body of the subject with light in a predetermined wavelength band. The time measurer measures an irradiation time of light in one treatment. The controller controls the irradiator so that the irradiation time is less than eight hours.

## Description

### TECHNICAL FIELD

The present disclosure relates to a light irradiation system.

### BACKGROUND OF INVENTION

Ganglion cells, such as intrinsically photosensitive retinal ganglion cells (iPRGCs), are present in the eyes, which can receive light but are not directly related to visual function.

Light reception by the intrinsically photosensitive retinal ganglion cells is known to produce various non-visual effects in mind and body. For example, irradiation with light in a wavelength band corresponding to blue light is known to suppress melatonin secretion in the pineal gland. Non-Patent Document 1 reports that exposure to light in a wavelength band corresponding to green light for at least eight hours alleviates acute and chronic pain.

### CITATION LIST

### NON-PATENT LITERATURE

Non-Patent Document 1: Mohab M. Ibrahima et, al., "Long-lasting antinociceptive effects of green light in acute and chronic pain in rats", Pain. Vol. 158, No. 2, p. 347-360, 2017.

### SUMMARY

A light irradiation system according to an aspect of the present disclosure includes an irradiator configured to irradiate a predetermined part of the body of a subject with irradiation light in a predetermined wavelength band, a time measurer configured to measure an irradiation time of the irradiation light in one treatment, and a controller configured to control the irradiator, and the controller controls the irradiation time to be less than eight hours.

### BRIEF DESCRIPTION OF THE DRAWINGS

FIG. 1 is an external view illustrating a configuration example of a light irradiation system according to a first embodiment of the present disclosure.
FIG. 2 is a block diagram illustrating an example of a configuration of main components of the light irradiation system according to the first embodiment of the present disclosure.
FIG. 3 is a flowchart illustrating a flow of processing executed by the light irradiation system according to the first embodiment of the present disclosure.
FIG. 4 is an external view illustrating a configuration example of a light irradiation system according to a second embodiment of the present disclosure.
FIG. 5 is a block diagram illustrating an example of a configuration of main components of the light irradiation system according to the second embodiment of the present disclosure.
FIG. 6 illustrates an example of a presentation screen presented on a presentator.
FIG. 7 illustrates an example of a presentation screen presented on the presentator.
FIG. 8 illustrates an example of treatment schedule information displayed on a screen of a communication device.
FIG. 9 is a perspective view illustrating a configuration example of a light irradiation system according to a third embodiment.
FIG. 10 is an external view illustrating a configuration example of a light irradiation system according to a fourth embodiment of the present disclosure.
FIG. 11 is a block diagram illustrating an example of a configuration of main components of the light irradiation system according to the fourth embodiment of the present disclosure.
FIG. 12 is a diagram illustrating an example of an irradiator in which multiple light emitting elements are arranged.
FIG. 13 is a diagram illustrating an example of the irradiator in which multiple light emitting elements are arranged.
FIG. 14 is a diagram illustrating examples of objects to be displayed on the irradiator.
FIG. 15 is a flowchart illustrating a flow of a control method executed by the light irradiation system according to the fourth embodiment of the present disclosure.
FIG. 16 is a block diagram illustrating an example of a configuration of main components of a light irradiation system according to a fifth embodiment of the present disclosure.
FIG. 17 is a flowchart illustrating a flow of a control method executed by the light irradiation system according to the fifth embodiment of the present disclosure.
FIG. 18 is a diagram illustrating a schedule of a light irradiation test.
FIG. 19 is a graph showing results of an analgesic test.
FIG. 20 is a graph showing results of blood enkephalin concentration analysis.

### DESCRIPTION OF EMBODIMENTS

### First Embodiment

An embodiment of the present disclosure will be described in detail below.

### Configuration of Light Irradiation System 100

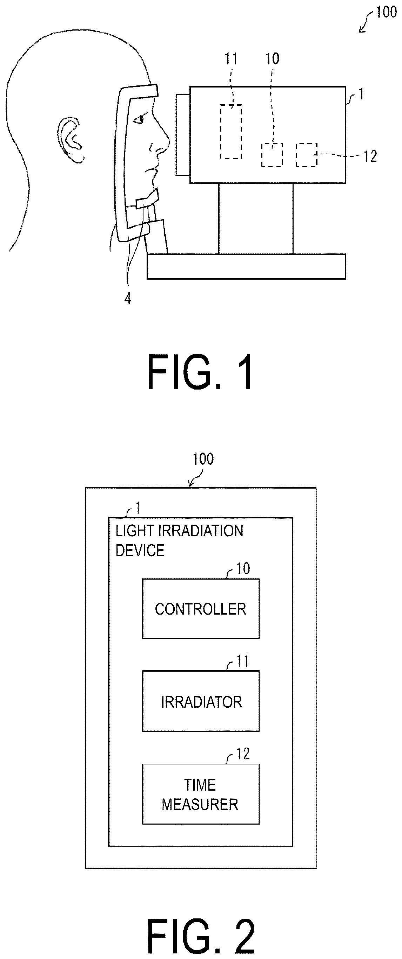

A configuration of a light irradiation system 100 according to an aspect of the present disclosure will be described with reference to FIGS. 1 and 2. FIG. 1 is a diagram illustrating a configuration example of the light irradiation system 100 according to an aspect of the present disclosure. FIG. 2 is a block diagram illustrating an example of a configuration of main components of the light irradiation system 100 according to a first embodiment of the present disclosure.

The light irradiation system 100 includes a light irradiation device 1, which is a device for irradiating a predetermined part of the body of a subject with irradiation light in a predetermined wavelength band. The light irradiation device 1 may be an installation-type device or may be a wearable device. In this specification, a case where a subject to be irradiated with irradiation light is a human being will be described as an example, but the subject is not limited to a human being. For example, various animals including dogs, cats, rabbits, guinea pigs, and rats can be subjects to be irradiated with irradiation light.

The predetermined part of the body of the subject to be irradiated with light may be any part of the body as long as a pain alleviation effect can be obtained. In the present disclosure, for example, the eyes of the subject are irradiated with light. The predetermined part to be irradiated with light may be, for example, the ear. When the ear of the subject is irradiated with light, the light irradiation device 1 may be, for example, an earphone-type device to be used by being inserted into the ear hole of the subject. The earphone-type light irradiation device 1 may direct light toward the eardrum through the external auditory canal of the subject.

In an aspect of the present disclosure, the light irradiation system 100 may irradiate both the eyes and the ears of the subject.

The light irradiation device 1 illustrated in FIG. 1 is, as an example, an installation type, and may include a fixing portion 4 for fixing the chin and the forehead of the subject to be irradiated with light. Since the face of the subject is fixed by the fixing portion 4, positions of the eyes of the subject are also fixed. In this case, the subject may be irradiated with light while sitting on a chair. The light irradiation device 1 illustrated in FIG. 1 can irradiate both the eyes of the subject with light in a predetermined wavelength band, but is not limited thereto. The light irradiation device 1 may irradiate one eye of the subject with light in a predetermined wavelength band.

The light irradiation device 1 illustrated in FIG. 1 may be installed in, for example, a medical institution, or may be installed in a home of the subject.

A configuration of the light irradiation device 1 will be described with reference to FIG. 2. As illustrated in FIG. 2, the light irradiation device 1 includes a controller 10, an irradiator 11, and a time measurer 12.

### Irradiator 11

The irradiator 11 includes one or more light sources for irradiating a predetermined part of the body of the subject with irradiation light in a predetermined wavelength band. The irradiator 11 may include, for example, a light emitting element such as a light emitting diode (LED) or a semiconductor laser (LD) as a light source. By using such a light emitting element, monochromatic light having a narrow emission wavelength width can be selectively emitted from the irradiator 11. Alternatively, the irradiator 11 may include a light source that emits white light and an optical filter (for example, a band-pass filter) that transmits only the light in a predetermined wavelength band. By selecting and using an appropriate optical filter, irradiation light in a desired wavelength band can be selectively emitted from the irradiator 11.

### Wavelength Band of Irradiation Light

The predetermined wavelength band of the irradiation light may be a wavelength band of light that is expected to improve mental and physical conditions of the subject by irradiating the eyes of the subject.

Irradiating the eyes of the subject with green light can continuously alleviate physical pain such as pain felt by the subject, after the irradiation. Therefore, the wavelength band of the irradiation light may be the wavelength band of green light. In this case, the predetermined wavelength band of the irradiation light is, for example, from 450 nm to 600 nm, and may be from 500 nm to 550 nm. The irradiation light may be green light with a peak top at 525 nm. For example, "from 450 nm to 600 nm" is intended to include wavelengths of 450 nm and 600 nm, and the same or similar notation applies to other wavelengths.

Irradiating the eyes of the subject with violet light can reduce deterioration of visual acuity of the subject or slow progression of myopia. Therefore, the wavelength band of the irradiation light may be the wavelength band of violet light. In this case, the predetermined wavelength band of the irradiation light may be from 350 nm to 410 nm.

The symptoms of jet lag in the subject can be alleviated by irradiating the eyes of the subject with blue light. Therefore, the wavelength band of the irradiation light may be the wavelength band of blue light. However, when the eyes of the subject are irradiated with blue light, an optical filter that blocks ultraviolet rays and near-ultraviolet rays may be further provided to reduce damage to the eyes of the subject as much as possible.

When an effect of improving the mental and physical conditions of the subject is expected, the irradiation light may be continuous light that is continuously emitted to the eyes of the subject, or pulsed light to be emitted intermittently.

### Time Measurer 12

The time measurer 12 measures an irradiation time in one treatment. The time measurer 12 may start time measurement from a time when the irradiator 11 starts light irradiation, and stop the time measurement when the light irradiation is stopped.

### Controller 10

The controller 10 controls the irradiator 11 so that the irradiation time in one treatment is less than eight hours. The controller 10 acquires the irradiation time from the time measurer 12. When the irradiation time reaches a predetermined treatment time of less than eight hours, the controller 10 stops the light irradiation by the irradiator 11. The predetermined treatment time may be set in advance, and may be set for each subject, for example.

### Irradiation Time

An upper limit of the irradiation time in one treatment may be 7.5 hours or less, more preferably 4 hours or less, even more preferably 2 hours or less, and most preferably 1 hour or less. According to this, a time required for the subject to receive treatment is reduced, and physical and mental strain on the subject is reduced.

A lower limit of the irradiation time in one treatment may be 30 minutes or more, and more preferably 1 hour or more. According to this, a pain alleviation effect can be given to the subject.

The light irradiation device 1 that irradiates the ear with irradiation light includes the controller 10, the irradiator 11, and the time measurer 12, as the same as and similar to the above-described light irradiation device 1 that irradiates the eyes with light. For example, when the light irradiation device 1 is an earphone-type, the irradiator 11 may be installed in the light irradiation device 1 on a side to be inserted into the ear hole, and light may be directed toward an inner part of the ear by the irradiator 11. The controller 10 and the time measurer 12 may be installed in the earphone-type light irradiation device 1 on the outside of the ear hole, which is a side opposite to the irradiator 11 side. The controller 10 controls the irradiator 11 so that the irradiation time is less than eight hours.

### Processing Executed by Light Irradiation System 100

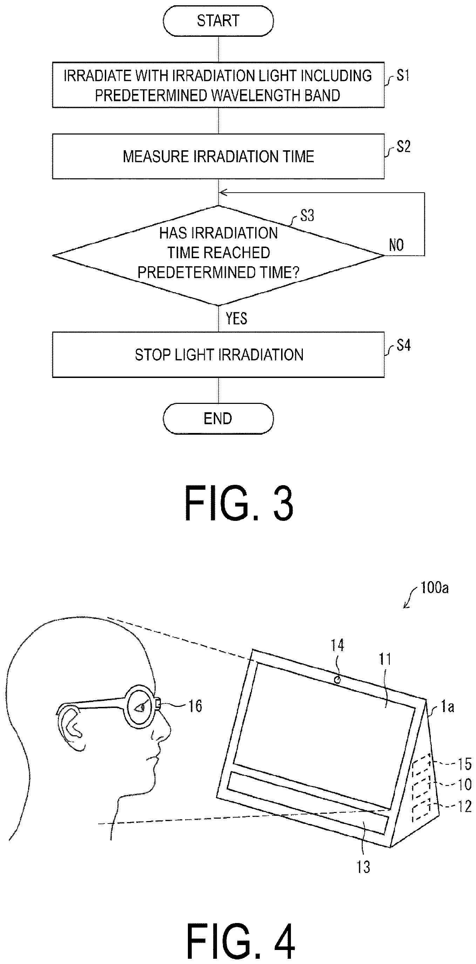

FIG. 3 is a flowchart illustrating a flow of processing executed by the light irradiation system 100 according to the present embodiment.

In S1, first, the irradiator 11 irradiates the eyes of the subject with irradiation light in a predetermined wavelength band (irradiation step).

In S2, the time measurer 12 starts measuring an irradiation time (time measurement step).

In S3, the controller 10 determines whether the irradiation time has reached the predetermined time of less than eight hours. When the controller 10 determines that the irradiation time has reached the predetermined time, the controller 10 executes the processing in S4. When the controller 10 determines that the irradiation time has not reached the predetermined time, the processing in S3 is repeated until the irradiation time is determined to have reached the predetermined time.

In S4, after the irradiation time was determined to have reached the predetermined time, the controller 10 causes the irradiator 11 to stop light irradiation (irradiation time control step).

According to the above-described processing, the irradiator 11 can irradiate irradiation light in a predetermined wavelength band, the time measurer 12 can measure an irradiation time, and the controller 10 can control the irradiation time to be less than eight hours. Thus, the eyes of the subject can be irradiated with light in the predetermined wavelength band for less than eight hours to provide the subject with a pain alleviation effect.

### Second Embodiment

Another embodiment of the present disclosure will be described below. For the sake of convenience of description, members having the same functions as those of the members described in the above-described embodiment are denoted by the same reference signs, and description thereof is not repeated.

In the first embodiment, the light irradiation system 100 is a light irradiation system in which an irradiation time for one treatment with irradiation light in a predetermined wavelength band is less than eight hours.

From the viewpoint of a pain alleviation effect, light irradiation performed in one treatment may be divided into multiple times and performed intermittently. The treatment of irradiating the eye of the subject with light may be performed two or more times. A frequency at which each treatment is performed is not limited, and the treatment may be performed once a day, or the treatment may be performed once on consecutive days. As an example, the treatment once a day may be performed for two to five consecutive days as one cycle. In this case, for example, a total treatment time to be performed in one cycle may be set in advance, and the treatment time per treatment may vary from day to day as long as the total treatment time is the set total treatment time. This one cycle may be performed regularly on a weekly basis or a monthly basis. The treatment does not need to be performed on consecutive days. For example, the treatment may be performed every other day, or the treatment may be performed for several hours per day over several consecutive days, followed by one day of no treatment, and then the treatment may be performed for several hours per day over several days. The treatment may be performed multiple times per day. For example, one-hour treatment may be performed twice a day. In an example in which the total treatment time is four hours, for example, one treatment for four hours may be continuously performed, or one treatment for one hour may be repeated four times with a rest time of one hour therebetween.

From the above viewpoint, in the present embodiment, the light irradiation system is a light irradiation system for repeatedly subjecting a predetermined part of the body of a subject to light irradiation treatment.

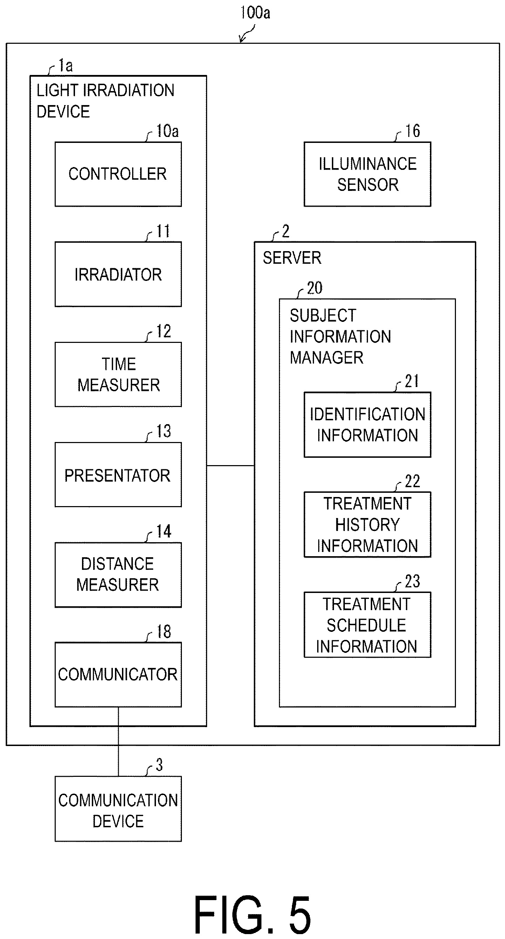

The present embodiment will be described with reference to FIGS. 4 and 5. FIG. 4 is an external view illustrating a configuration example of a light irradiation system according to a second embodiment. FIG. 5 is a block diagram illustrating a configuration example of the light irradiation system according to the second embodiment.

As illustrated in FIG. 4, a light irradiation system 100a of the present embodiment includes, as an example, an installation-type light irradiation device 1a. For example, a subject receives treatment while facing the light irradiation device 1a. At this time, the subject can receive the treatment while sitting on a chair, for example. The light irradiation device 1a includes an irradiator 11, and as an example, includes a presentator 13 below the irradiator 11. As illustrated in FIG. 4, the irradiator 11 may be located on one surface of the light irradiation device 1a. The light irradiation device 1a may include a distance measurer 14 that measures a distance between the subject and the irradiator 11. The light irradiation system 100a includes, as an example, an illuminance sensor 16.

As illustrated in FIG. 5, the light irradiation system 100a of the present embodiment may further include a server 2 and the illuminance sensor 16 in addition to the light irradiation device 1a. The server 2 may include a subject information manager 20. The light irradiation device 1a includes the presentator 13, the distance measurer 14, and a communicator 18 in addition to a controller 10a, the irradiator 11, and the time measurer 12.

### Server 2

As an example, the server 2 is communicably connected to the light irradiation device 1. The server 2 may be a server owned by a medical institution, for example. The server 2 includes the subject information manager 20.

### Subject Information Manager 20

The subject information manager 20 manages identification information 21 about the subject, treatment history information 22, and treatment schedule information 23 in association with each other for each subject. The identification information 21 about the subject is information to be given to each subject in order to identify the subject, and is composed of, for example, a numeric string or a character string. The treatment history information 22 is information that includes an irradiation time in one treatment received by the subject and the number of times of the treatment received by the subject. The treatment history information 22 may include, for example, a date and time at which the treatment was performed, and may include a time when one treatment was started and a time when one treatment was finished. The treatment history information 22 may include a total cumulative irradiation time obtained by multiplying the number of consecutive days the subject has received the treatment by the irradiation time for one treatment. The treatment schedule information 23 is information about a treatment schedule that the subject is scheduled to receive. The treatment schedule information 23 may be, for example, a treatment start time and a treatment end time on the next day, or may be a treatment start date and a treatment end date of the next week or the next month, which is the next treatment cycle.

The light irradiation device 1a may receive stored information from the subject information manager 20 as necessary.

As described above, by associating the identification information 21 about the subject, the treatment history information 22, and the treatment schedule information 23 with each other for each subject, the subject information manager 20 can manage the treatment history information 22 and the treatment schedule information 23 for each subject.

### Presentator 13

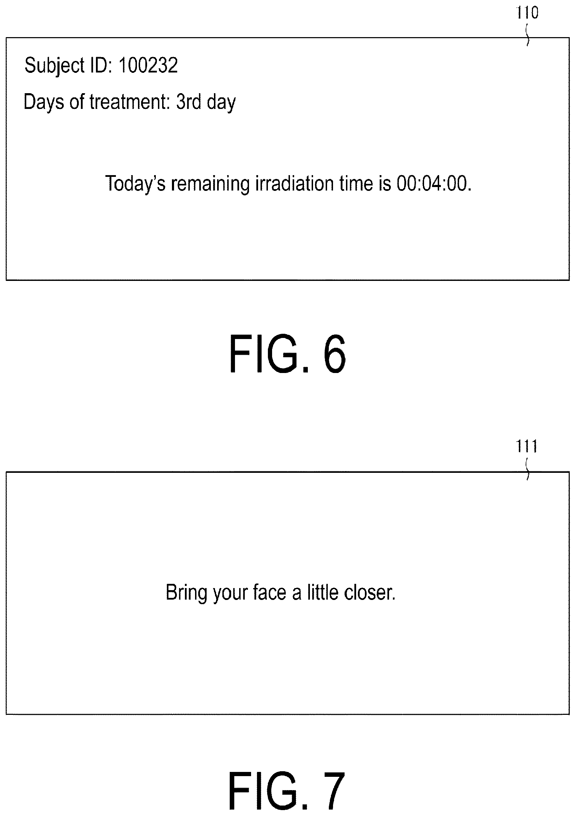

The presentator 13 presents the treatment history information 22 to the subject information. The presentator 13 may be, for example, a display for displaying letters or a speaker for emitting sound. FIG. 6 illustrates an example of a presentation screen 110 to be presented on the presentator 13, where the presentator 13 is a display. The presentation screen 110 may display "subject ID" as the identification information 21, and "days of treatment" as the treatment history information 22. The presentator 13 may present, along with the treatment history information 22, information about a scheduled end time of the treatment currently being received. The information about the scheduled end time may be a scheduled end time or may be a remaining time until the scheduled end time. For example, the presentation screen 110 to be presented on the presentator 13 may show the time remaining until the scheduled end time of the treatment currently being received, such as "Today's remaining irradiation time is 00:04:00".

### Distance Measurer 14

The distance measurer 14 measures a distance between the irradiator 11 and the eye of the subject. In order to obtain a pain alleviation effect, the eye of the subject is required to be irradiated with light with a predetermined illuminance value. This requires an appropriate distance between the irradiator 11 and the eye of the subject. The distance measurer 14 is not limited as long as it can measure the distance between the irradiator 11 and the eye of the subject. For example, the distance measurer 14 is an infrared camera. With the infrared camera, the distance between the irradiator 11 and the subject eye can be measured.

Based on a result by the distance measurer 14, the presentator 13 may present a presentation screen for instructing the subject to move a position of a predetermined part of the body. For example, when irradiating the eye of the subject, the presentator 13 may present a presentation screen instructing the subject to change a position of the head or face in order to appropriately adjust the distance between the irradiator 11 and the eye of the subject. Alternatively, the presentator 13 may present a presentation screen instructing the subject to change posture in order to appropriately adjust the distance between the irradiator 11 and the eye of the subject. The controller 10 may control the presentator 13 to present the presentation screen. FIG. 7 illustrates an example of a presentation screen 111 to be presented on the presentator 13, where the presentator 13 is a display. As an example, the presentation screen 111 presents a message "Bring your face a little closer". As described above, by presenting the presentation screen for instructing the subject to move the position of the predetermined part of the body, the presentator 13 can, for example, prompt the subject to bring the face closer to the irradiator 11. According to this, the distance between the irradiator 11 and the eye of the subject is appropriately adjusted, and the irradiator 11 can irradiate the eye of the subject with light with an appropriate illuminance value.

### Communicator 18

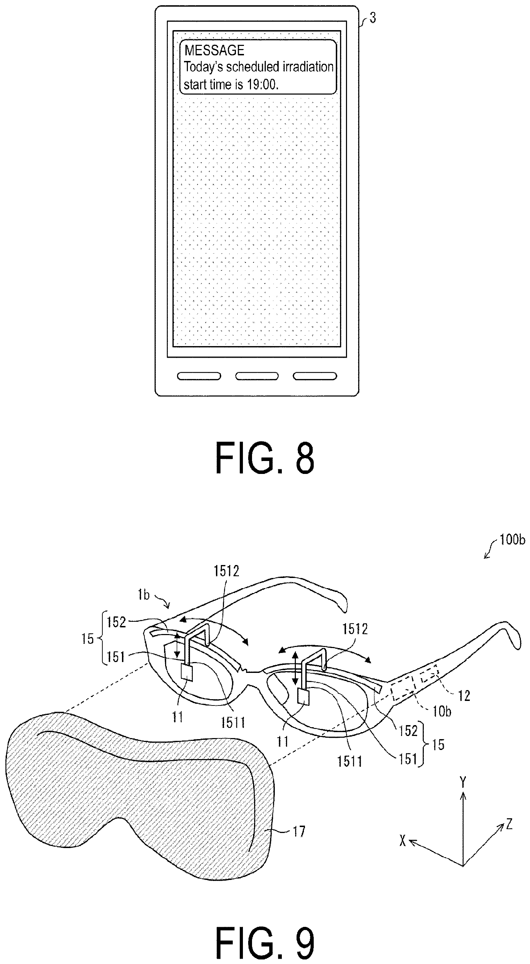

The communicator 18 communicates with a communication device 3 outside the light irradiation device 1. The communication device 3 is not limited as long as it has a function of communicating with the light irradiation device 1. For example, the communication device 3 may be a PC, a tablet, a smartphone, or the like. The communication device 3 may be a PC having a function of directly communicating only with the light irradiation device 1. For example, the communication device 3 may be a communication device 3 owned by the subject. The communicator 18 may transmit the treatment schedule information 23 managed by the subject information manager 20 to the communication device 3 of the subject corresponding to this treatment schedule information. FIG. 8 is an example of the treatment schedule information 23 transmitted from the communicator 18 and to be displayed on the screen of the communication device 3 of the subject. The communication device 3 is, as an example, a smartphone owned by the subject. On the screen of this smartphone, information for notifying the subject of a treatment schedule is displayed as the treatment schedule information 23. As an example, the screen of the smartphone displays a message "Today's scheduled irradiation start time is 19:00". The communicator 18 transmits the treatment schedule information 23 to the communication device of the subject, so the subject can know the treatment schedule and can remember to receive the treatment.

### Illuminance Sensor 16

The illuminance sensor 16 is a sensor that detects illuminance. As described in Distance Measurer 14, in order to obtain a pain alleviation effect, the eye of the subject is required to be irradiated with light with a predetermined illuminance value. Therefore, the illuminance sensor 16 may be attached to a place close to the eye of the subject to detect the illuminance value of the place close to the eye. In FIG. 4, as an example, the illuminance sensor 16 is attached to a frame of glasses. The manner in which the illuminance sensor 16 is attached is not limited to this. For example, the illuminance sensor 16 may be attached directly to the skin of the subject, or the subject may wear a cap to which the illuminance sensor 16 is attached.

The distance measurer 14 may measure the distance between the irradiator 11 and the eye of the subject based on the detected illuminance value detected by the illuminance sensor 16. Accordingly, the distance between the irradiator 11 and the eye of the subject can be measured.

The controller 10 may adjust the output of the irradiator 11 based on the detected illuminance value detected by the illuminance sensor 16. Thus, the eye of the subject is irradiated with light with an appropriate illuminance value.

### Third Embodiment

Another embodiment of the present disclosure will be described below. For the sake of convenience of description, members having the same functions as those of the members described in the above-described embodiments are denoted by the same reference signs, and description thereof is not repeated.

In the first and second embodiments, the light irradiation system including the installation-type light irradiation device has been described. In the present embodiment, a light irradiation system 100b including a wearable light irradiation device will be described.

The present embodiment will be described with reference to FIG. 9. FIG. 9 is a perspective view illustrating a configuration example of a light irradiation system 100b according to a third embodiment.

The light irradiation system 100b includes a light irradiation device 1b. The light irradiation device 1b includes a shielding mechanism 17 in addition to a controller 10b, an irradiator 11, and a time measurer 12. The light irradiation device 1b may include a moving mechanism 15.

### Shielding Mechanism 17

The shielding mechanism 17 may reduce illuminance of light different from irradiation light, or illuminance of light in a wavelength band different from a predetermined wavelength band that is included in light different from the irradiation light. As used herein, the light different from the irradiation light may be, for example, sunlight or light from indoor lighting.

The shielding mechanism 17 may be made of fabric or resin having light shielding properties, or metal having light shielding properties or light reflecting properties. Alternatively, the shielding mechanism 17 may include an optical filter that selectively transmits light in a predetermined wavelength band and does not transmit light in a wavelength band different from the predetermined wavelength band. When the shielding mechanism 17 is made of a light shielding material, the shielding mechanism 17 can reduce illuminance of light from the outside of the light irradiation device 1b over an entire wavelength band. When the shielding mechanism 17 is made of a material that selectively transmits light in a predetermined wavelength band and does not transmit light in a wavelength band different from the predetermined wavelength band, the shielding mechanism 17 can reduce illuminance of light in the wavelength band different from the predetermined wavelength band that is included in the light from the outside of the light irradiation device 1b. Thus, the shielding mechanism 17 can reduce the illuminance of light in the wavelength band different from the wavelength band of the irradiation light reaching the eyes of the subject when the eyes of the subject are irradiated with the irradiation light in the predetermined wavelength band. When the shielding mechanism 17 is made of a light reflecting material, the light from the outside of the light irradiation device 1b can be reduced, and the light inside the light irradiation device 1b can be reflected to the subject to be irradiated.

The shielding mechanism 17 may be positioned in a manner that no gap exists between the subject wearing the light irradiation device 1b and the light irradiation device 1b. The shielding mechanism 17 may be integrated with or may be separated from the light irradiation device 1b.

As illustrated in FIG. 9, the shielding mechanism 17 may have a goggle-like shape covering an entire frame portion of the light irradiation device 1b. In this case, the shielding mechanism 17 can cover the frame portion of the light irradiation device 1b. This avoids that light from the outside of the light irradiation device 1b enters through gaps between the forehead, the temples, the cheeks, and the nose of the subject and the light irradiation device 1b, and reaches the eyes of the subject. Alternatively, the shielding mechanism 17 may cover the entire head of the subject. The subject wearing the light irradiation device 1b illustrated in FIG. 9 or the head and other parts of the subject may be covered with another shielding mechanism 17.

The shielding mechanism 17 illustrated in FIG. 9 shields light reaching both the eyes of the subject from the outside of the light irradiation device 1b, but is not limited thereto. For example, the light irradiation device 1b may include a shielding mechanism 17 for the right eye and a shielding mechanism 17 for the left eye. In this case, when one eye of the subject is irradiated with the irradiation light, only the shielding mechanism 17 on the same side may be used. With this configuration, the field of view on the side not irradiated with the irradiation light is not blocked by the shielding mechanism 17. This allows the subject to visually recognize a surrounding situation with the eye on the side not irradiated with the irradiation light.

### Moving Mechanism 15

The light irradiation device 1b may include the moving mechanism 15 for adjusting a position of the irradiator 11. The moving mechanism 15 is provided to move the position of the irradiator 11 so that light from the irradiator 11 can be directed toward the eye of the subject, in particular the pupil.

The moving mechanism 15 may have any configuration with which the position of the irradiator 11 can be changed with respect to the position of the pupil of the eye of the subject. As an example, as illustrated in FIG. 9, the moving mechanism 15 may include a holding portion 151 that holds the irradiator 11 at a first end 1511, and a guide rail 152 for moving the holding portion 151 in the X-axis direction (horizontal direction).

The guide rail 152 may be, for example, a groove formed in an upper portion of the frame of the light irradiation device 1b. A second end 1512 of the holding portion 151 opposite to the first end 1511 may slidably abut an inner surface of the groove. Thus, the holding portion 151 can move in the X-axis direction along the guide rail 152.

The moving mechanism 15 may move the position of the irradiator 11 in the Y-axis direction (vertical direction). For example, the holding portion 151 may have a structure expandable and contractable in the Y-axis direction. Alternatively, a fixing member (not illustrated) that fixes the irradiator 11 to the holding portion 151 may be provided slidably along the holding portion 151 along which the irradiator 11 can move.

For example, medical personnel such as a doctor and a nurse in charge of the subject (or the subject himself/herself) can manually change the position of the irradiator 11 along the guide rail 152.

With the moving mechanism 15, the light irradiation device 1b can accurately irradiate the eye of the subject, particularly the pupil, with the irradiation light and cause the irradiation light to reach the central fovea of the retina.

### Conclusion

According to an aspect 1A of the present invention, a light irradiation system includes an irradiator configured to irradiate a predetermined part of the body of a subject with irradiation light in a predetermined wavelength band, a time measurer configured to measure an irradiation time of the irradiation light in one treatment, and a controller configured to control the irradiator, and the controller controls the irradiation time to be less than eight hours.

In the light irradiation system according to an aspect 2A of the present invention, in the aspect 1A, the predetermined part of the body of the subject may be the eye and/or the ear.

In the light irradiation system according to an aspect 3A of the present invention, in the aspect 1A or 2A, a length of time of the treatment may be 0.5 hours or more.

In the light irradiation system according to an aspect 4A of the present invention, in any one of the aspects 1A to 3A, a length of time of the treatment may be four hours or less.

In the light irradiation system according to an aspect 5A of the present invention, in any one of the aspects 1A to 3A, a length of time of the treatment may be two hours or less.

In the light irradiation system according to an aspect 6A of the present invention, in any one of the aspects 1A to 3A, a length of time of the treatment may be one hour or less.

In the light irradiation system according to an aspect 7A of the present invention, in any one of the aspects 1A to 3A, a length of time of the treatment may be 0.5 hours.

According to an aspect 8A of the present invention, in any one of the aspects 1A to 7A, the light irradiation system may further include a subject information manager configured to manage identification information of the subject and treatment history information including the irradiation time of the treatment and the number of times of the treatment received by the subject in association with each other subject by subject, and a presentator configured to present the treatment history information to the subject corresponding to the treatment history information.

In the light irradiation system according to an aspect 9A of the present invention, in the aspect 8A, the presentator may present to each subject a scheduled end time of the treatment currently being given to the subject.

According to an aspect 10A of the present invention, in the aspect 8A or 9A, the light irradiation system may include a communicator configured to communicate with a communication device of the subject, and the subject information manager may manage the identification information about the subject, the treatment history information including the irradiation time and the number of times of the treatment received by the subject, and treatment schedule information about a schedule of the treatment to be received by the subject in association with each other, and the treatment schedule information is transmitted to the communication device of the subject corresponding to the treatment schedule information.

According to an aspect 11A of the present invention, in any one of the aspects 8A to 10A, the light irradiation system may further include a distance measurer configured to measure a distance between the irradiator and the eye of the subject, and the presentator may present a presentation screen configured to instruct the subject to move a position of the predetermined part of the body according to the distance.

According to an aspect 12A of the present invention, in the aspect 11A, the light irradiation system may include an illuminance sensor configured to be attached to the subject, and the distance measurer may measure the distance between the irradiator and the eye of the subject based on an illuminance value detected by the illuminance sensor.

According to an aspect 13A of the present invention, in the aspect 11A or 12A, the light irradiation system may include an illuminance sensor configured to be attached to the subject, and the controller may adjust an output of irradiation light by the irradiator based on an illuminance value detected by the illuminance sensor.

According to an aspect 14A of the present invention, in any one of the aspects 1A to 13A, the light irradiation system may further include a shielding mechanism configured to reduce illuminance of light different from the irradiation light and at least in a wavelength band different from the predetermined wavelength band.

According to an aspect 15A of the present invention, a light irradiation device includes an irradiator configured to irradiate a predetermined part of the body of a subject with irradiation light in a predetermined wavelength band, a time measurer configured to measure an irradiation time of the irradiation light in one treatment, and a controller configured to control the irradiator, and the controller controls the irradiation time to be less than eight hours.

According to an aspect 16A of the present invention, a method for controlling a light irradiation system includes irradiating a predetermined part of the body of a subject with irradiation light in a predetermined wavelength band, measuring an irradiation time of the irradiation light in one treatment, and controlling the irradiation time to be less than eight hours.

### Fourth Embodiment

### Configuration of Light Irradiation System 100c

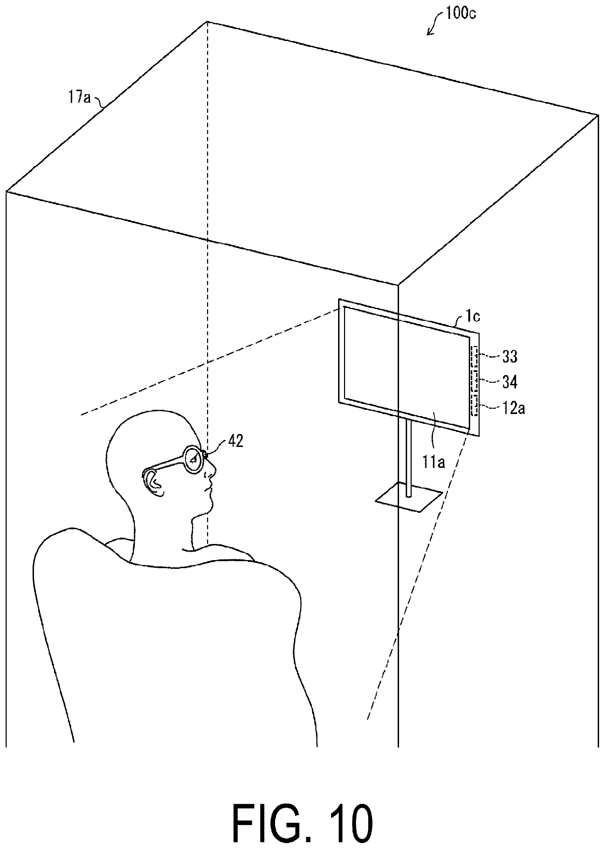

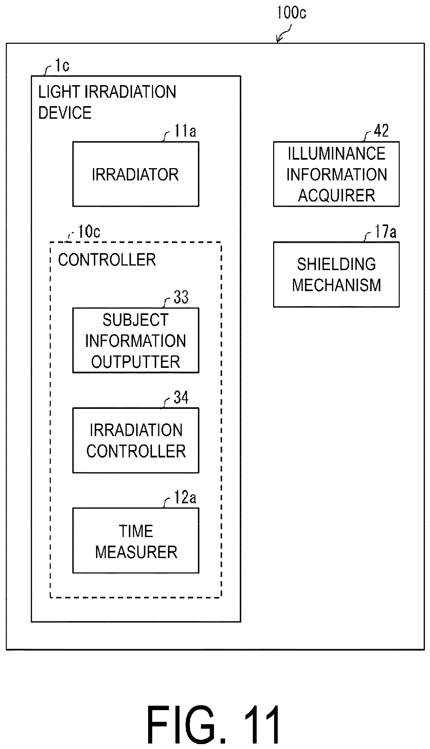

In an aspect of the present disclosure, a configuration of a light irradiation system 100c will be described with reference to FIGS. 10 and 11. FIG. 10 is an external view illustrating a configuration example of the light irradiation system 100c according to an aspect of the present disclosure. FIG. 11 is a block diagram illustrating an example of a configuration of main components of the light irradiation system 100c according to a fourth embodiment of the present disclosure.

The light irradiation system 100c includes a light irradiation device 1c, which is a device for irradiating the eyes of a subject with light in a predetermined wavelength band. The light irradiation device 1c may be an installation-type device. The light irradiation device 1c in the light irradiation system 100c illustrated in FIG. 10 is, as an example, an installation-type device.

For example, the subject may be irradiated with light in a predetermined wavelength band while sitting facing the light irradiation device 1c. Hereinafter, irradiating a subject with light is also referred to as "treatment" or "medical treatment". The subject may wear glasses provided with an illuminance information acquirer 42. According to this, the illuminance information acquirer 42 can acquire illuminance information indicating an illuminance value in the vicinity of the eye of the subject by light to be emitted from the light irradiation device 1. The illuminance information acquirer 42 may be provided in a device that can be worn by the subject, as described above, or may be provided in a chair on which the subject sits. The illuminance value in the vicinity of the eye of the subject may be obtained.

Illuminance of light with which the eye of the subject is irradiated depends on, for example, a position where the subject sits, posture of the subject, and the like. The illuminance value of light is less than a predetermined illuminance value when the subject wears the glasses provided with the illuminance information acquirer 42, as described above, for example, because the subject sits deeply on a backrest and is far from the irradiator 11, or the subject is looking down. When the illuminance value of the light is less than the predetermined illuminance value, a subject information outputter 33 outputs information for prompting a change in a state of the subject. The subject information outputter 33 will be described in detail below.

The light irradiation system 100c illustrated in FIG. 10 may be installed in, for example, a medical institution or a home of the subject.

A configuration of the light irradiation system 100c will be described with reference to FIG. 11. As illustrated in FIG. 2, the light irradiation system 100c includes the light irradiation device 1c, the illuminance information acquirer 42, and a shielding mechanism 17a.

### Illuminance Information Acquirer 42

The illuminance information acquirer 42 is positioned to face the irradiator 11a and acquires illuminance information indicating an illuminance value of light. Here, the illuminance information may be a measured value obtained by measuring an illuminance value, or may be a determination result obtained by determining whether the measured illuminance value is within a predetermined range. Alternatively, the illuminance information may be an estimated value obtained by estimating an illuminance value of light reaching the retina of the subject based on the measured illuminance value. The illuminance information acquirer 42 may be, for example, an illuminance sensor. A type of illuminance sensor is not limited. The illuminance sensor may be a sensor using a phototransistor, a sensor using photodiode, or a sensor obtained by adding an amplifier circuit to a photodiode.

### Light Irradiation Device 1c

The light irradiation device 1c includes the irradiator 11a and a controller 10c. The controller 10c includes the subject information outputter 33, an irradiation controller 34, and a time measurer 12a.

### Irradiator 11a

The irradiator 11a irradiates the eyes of a subject with irradiation light in a predetermined wavelength band. The irradiator 11a may include multiple light emitting elements that emit light. The multiple light emitting elements may be, for example, light emitting elements such as light emitting diodes (LEDs) or semiconductor lasers (LDs). By using such light emitting elements, monochromatic light having a narrow emission wavelength width can be selectively irradiated by the irradiator 11a.

### Light Emitting Elements in Irradiator 11a

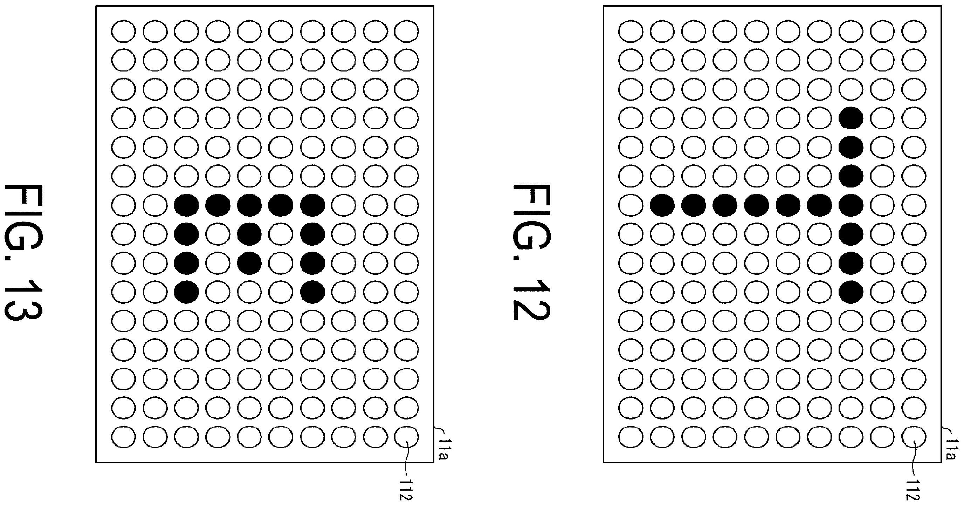

The irradiator 11a may include the multiple light emitting elements each capable of emitting light of a predetermined wavelength, and the multiple light emitting elements may be arranged. FIG. 12 is a diagram illustrating an example of the irradiator 11a in which the multiple light emitting elements 112 are arranged. Although 150 light emitting elements are arranged in the irradiator 11a in FIG. 12 as an example, the number of light emitting elements is not limited thereto. The multiple light emitting elements may be light emitting elements of the same single color, and for example, only green light emitting elements may be arranged. Red, green, and blue light emitting elements may be arranged. According to this, light in a predetermined wavelength band can be emitted. Control of causing the light emitting element to emit light will be described in the description of Irradiation Controller 34.

### Illuminance of Light

A predetermined illuminance value of light to be emitted by the irradiator 11c may be from 4 to 110 Lux. When the illuminance value of light is within the above range, a pain alleviation effect can be given to the subject.

### Subject Information Outputter 33

When the illuminance value of light is less than the predetermined illuminance value, the subject information outputter 33 outputs information for prompting a change in the state of the subject. The information for prompting the change of the state of the subject is information for directing the direction of the face of the subject toward the irradiator 11a or information for bringing the face of the subject closer to the irradiator 11a. The subject information outputter 33 may be a speaker that outputs sounds such as alarms, announcements, and music, or may be a display that displays letters, numbers, pictures, and the like.

When the subject information outputter 33 is a speaker and the illuminance value of light is less than the predetermined value, a warning sound or an alarm may be output. The subject information outputter 33 may output voice announcements such as "Light is not properly emitted", "Raise your face", "Straighten your back", and "Bring your face closer". When the illuminance value of light fluctuates below and above the predetermined illuminance value, the subject may be asleep, so the subject information outputter 33 may output an announcement of "Wake up" or an alarm sound. According to this, the subject can know that the light is not properly emitted, and can correct posture or move the face closer so that the light is properly emitted. In a case where the subject information outputter 33 is a speaker, music may be output when the illuminance value of light is equal to or greater than the predetermined value, and playback of the music may be interrupted when the illuminance value of light is less than the predetermined value. According to this, the subject can be conscious of correcting the posture or facing the irradiator 1 1a so as not to interrupt the playback of the music.

When the subject information outputter 33 is a display and the illuminance value of light is less than the predetermined value, for example, letters such as "Light is not properly emitted", "Raise your face", "Straighten your back", or "Bring your face closer" may be displayed. The subject information outputter 33 may display a picture together with the letters. The light irradiation device 1c may include both the speaker and the display described above as the subject information outputter 33.

A timing at which the subject information outputter 33 outputs the information may be, for example, when a time below the predetermined illuminance value has lasted for several tens of seconds. The time below the predetermined illuminance value may be measured by, for example, the time measurer 12a described below. The subject information outputter 33 may acquire, for example, information that the illuminance value of light is less than the predetermined illuminance value from the illuminance information acquirer 42 described above. In this case, the subject information outputter 33 and the illuminance information acquirer 42 may be communicably connected by a wireless communication function or a wired communication function.

FIGS. 10 and 11 illustrate a configuration in which the light irradiation device 1c includes the subject information outputter 33 and outputs information prompting a change of the subject, but the configuration is not limited thereto. For example, a device other than the light irradiation device 1c may output information prompting the change in the state of the subject. For example, the glasses worn by the subject in FIG. 10 may include the subject information outputter 33. In this case, the subject information outputter 33 may output information prompting the change in the state of the subject by sound and vibration. According to this, the subject information outputter 33 can output the information for prompting the change in the state of the subject at a place close to the subject, so the subject can draw attention to this information.

It is assumed that a subject receives treatment in a private room and medical personnel are outside the private room. The subject may be unaware of an output from the subject information outputter 33 or may have fallen sleep. The information prompting the change in the state of the subject may also be output from the communication devices used by the medical personnel. In this case, the light irradiation device 1c may transmit, to communication devices used by the medical personnel, an output instruction instructing output of information prompting a change in the state of the subject. According to this, the medical personnel can receive the information prompting the change in the state of the subject. Even when the subject is unaware of the output from the subject information outputter 33, the medical personnel can directly prompt the subject to change the state of the subject.

### Irradiation Controller 34

The irradiation controller 34 may adjust intensity of the light emitted from the irradiator 11a based on the illuminance information. For example, the irradiation controller 34 may increase the intensity of the light emitted from the irradiator 11a based on the illuminance information indicating that the illuminance value is less than the predetermined illuminance value. When the illuminance value is less than the predetermined illuminance value even though the irradiation controller 34 increases the intensity of the light emitted from the irradiator 11a, information may be output from the subject information outputter 33.

Before starting treatment, the illuminance information may be acquired by having the subject sit in a predetermined position, and the irradiation controller 34 may determine the intensity of the light to be emitted from the irradiator 11a based on the acquired illuminance information. Before the irradiation controller 34 determines the intensity of light, the subject information outputter 33 may output information to prompt a change in the angle, height, or the like of the light irradiation device 1c.

Intensity of light depends on the number of light emitting elements that emit light and the magnitude of current applied to the light emitting elements. The irradiation controller 34 may cause at least one of the multiple light emitting elements included in the irradiator 11a to emit light. Thus, the irradiation controller 34 causes the light emitting elements to emit light, so that light can be emitted from the irradiator 11a.

The irradiation controller 34 may adjust the intensity of the light emitted from the irradiator 11a by controlling the number of the arranged light emitting elements (hereinafter, also simply referred to as "elements"), each of the light emitting elements being caused to emit light. Control of the number of elements that emit light executed by the irradiation controller 34 will be described with reference to FIG. 12, which is also described in Light Emitting Elements of Irradiator 11a. Among the multiple light emitting elements 112 arranged in the irradiator 11a in FIG. 12, as an example, light emitting elements 112 illustrated in white are elements that emit light, and light emitting elements 112 illustrated in black are elements that do not emit light. For example, when a predetermined illuminance value can be ensured by emitting light from 137 elements among the 150 elements arranged in the irradiator 11a, the irradiation controller 34 may cause the 137 elements to emit light as illustrated in FIG. 12. By controlling the number of light emitting elements that are caused to emit light by the irradiation controller 34 in this manner, the intensity of light emitted from the irradiator 11a can be adjusted. Thus, the predetermined illuminance value can be ensured. The irradiation controller 34 may also adjust the intensity of the light emitted from the irradiator 11a by changing the magnitude of the current applied to the light emitting elements.

The irradiation controller 34 may display an object composed of elements that emit light and elements that do not emit light on the irradiator 11a by controlling arrangement of the light emitting elements, each of the light emitting elements being caused to emit light among the multiple light emitting elements arranged. The object is not limited and may be, for example, letters, numbers, pictures, and the like. In FIG. 12, as an example, the letter "T" of the alphabet is displayed by the light emitting elements 112 that are not emitting light.

The object to be displayed on the irradiator 11a may be different for each unit time. FIG. 13 is a diagram illustrating an example of the irradiator 11a in which the multiple light emitting elements 112 are arranged. In the irradiator 11a in FIG. 13, 150 light emitting elements 112 are arranged as the same as and/or similar to the irradiator 11a in FIG. 12. As illustrated in FIG. 13, on the irradiator 11a, the letter "E" of the alphabet is displayed by the light emitting elements 112 that are not emitting light. For example, after the letter "T" is displayed as in the irradiator 11a in FIG. 12, the letter "E" may be displayed as in the irradiator 11a in FIG. 13. Thus, the irradiation controller 34 controls arrangement of the light emitting elements, each of the light emitting elements being caused to emit light among the multiple light emitting elements arranged, so that the object can be displayed on the irradiator 11a. The irradiator 11a not only irradiates with light but also displays objects, thereby maintaining concentration of the subject to be treated.

Even when the object to be displayed on the irradiator 11a is changed for each unit time, the number of light emitting elements 112 that emit light may be controlled by the irradiation controller 34. Here, the objects displayed on the irradiator 11a in FIG. 12 and the irradiator 11a in FIG. 13 are different, but the number of light emitting elements 112 emitting light is 137 in both cases. Thus, the irradiation controller 34 controls the number of light emitting elements 112 that emit light, thereby maintaining the predetermined illuminance value during the treatment.

### Example of Object

The object displayed on the irradiator 11a by the irradiation controller 34 is not limited, but may be a still image, a moving image, a message, or the like. The message may include, for example, letters, numbers, and symbols. Various objects can be applied to maintain the concentration of the subject during the treatment and to relax the subject during the treatment.

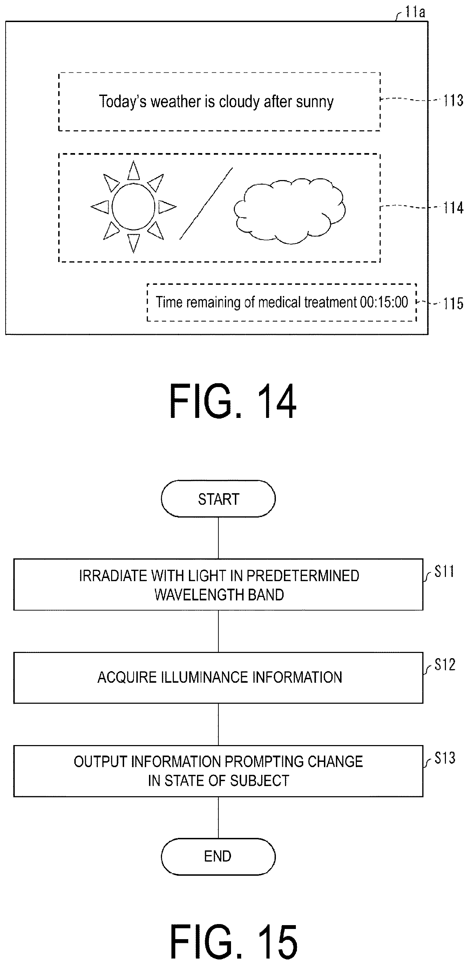

FIG. 14 is examples of objects to be displayed on the irradiator 11a. On the irradiator 11a in FIG. 14, as the examples, an object 113 representing the weather in letters, an object 114 representing the weather in pictures, and an object 115 representing a remaining treatment time are displayed. These letters, pictures, and numbers may be represented by elements that emit light and elements that do not emit light among the multiple light emitting elements 112, as described above. From the viewpoint of irradiating the eyes of the subject with sufficient light, for example, the letters, pictures, and numbers in FIG. 14 may be represented by elements that do not emit light, and the background of the letters, pictures, and numbers may be represented by elements that emit light. When a ratio of the object to be displayed is large with respect to an area of the irradiator 11a, the elements representing the object may be caused to emit light and the elements representing the background may not be caused to emit light. Thus, the predetermined illuminance value can be maintained.

The object displayed on the irradiator 11a by the irradiation controller 34 may be, for example, a news text, a novel text, or an animation. These objects may be changed for each unit time. The subject may be allowed to select which object to display prior to treatment. According to this, the attention of the subject can be drawn and the face of the subject can be naturally directed toward the irradiator 11a. The subject can be also relaxed. Sound, music, or the like that matches these objects may be played from speakers.

Since the irradiator 11a in the present disclosure may be, for example, a display capable of displaying letters, pictures, numbers, and the like, as described above, the irradiator 11a may display information prompting a change in the state of the subject on the irradiator 11a.

Regardless of what kind of object is to be displayed on the irradiator 11a, the irradiation controller 34 may control a ratio of elements that emit light and elements that do not emit light, so that light with an illuminance value equal to or greater than the predetermined illuminance value reaches the eyes of the subject. According to this, the eyes of the subject can be stably irradiated with light in a predetermined wavelength band.

### Time Measurer 12a

In the present embodiment, the irradiation time is a time during which the illuminance value of the light is equal to or greater than the predetermined illuminance value, and the time measurer 12a measures the time during which the illuminance value of the light is equal to or greater than the predetermined illuminance value as the irradiation time based on the illuminance information. The time measurer 12a may be, for example, a timer. For example, the time measurer 12a may start measuring time from a time when light irradiation by the irradiator 11a is started. The time measurer 12a may measure a time only when the illuminance value of light is equal to or greater than the predetermined illuminance value and may not measure a time when the illuminance value of light is less than the predetermined illuminance value. The time measurer 12a may separately measure a time when the illuminance value of light is equal to or greater than the predetermined illuminance value and a time when the illuminance value of light is less than the predetermined illuminance value. The time when the illuminance value of light is equal to or greater than the predetermined illuminance value may be stored in a subject database (not illustrated) for each subject, for example, as a cumulative time. According to this, the irradiation time can be managed for each subject.

The time measurer 12a may end irradiation by the irradiator 11a when a length of the irradiation time reaches a predetermined time. The predetermined time will be described later in Predetermined Time of Irradiation. At the end of the treatment, in order to notify the subject of the end of the treatment, the subject information outputter 33 may announce the end of the treatment, or the controller 10c may turn on lighting in the room. According to this, the subject does not need to measure the time himself/herself, and the light is emitted for a sufficient time for a pain alleviation effect.

### Predetermined Time of Irradiation

The length of the predetermined time during which the light is emitted may be 0.5 hours or more and less than 8 hours. An upper limit of the predetermined time may be 7.5 hours or less, 4 hours or less, 2 hours or less, or 1 hour or less. According to this, the time required for the treatment of irradiating the subject with light is reduced, and physical and mental strain on the subject is reduced.

A lower limit of the length of the predetermined time may be 30 minutes or more, or may be 1 hour or more. According to this, a pain alleviation effect can be given to the subject.

### Shielding Mechanism 17a

The shielding mechanism 17a may reduce illuminance of light at least in a wavelength band different from a predetermined wavelength band that is included in light different from the light emitted by the light irradiation device 1c. As used herein, the light different from the irradiation light may be, for example, sunlight or light from indoor lighting.

The shielding mechanism 17a may be made of fabric or resin having light shielding properties, or metal having light shielding properties or light reflecting properties. According to this, the shielding mechanism 17a can reduce the illuminance of light from the outside of the light irradiation device 1 over an entire wavelength band. Here, the shielding mechanism 17a may be specifically a curtain or a dark room. When lighting in the room can be turned off, the shielding mechanism is not necessarily provided.

### Control Method Executed by Light Irradiation System 100c

FIG. 15 is a flowchart illustrating a flow of a control method to be executed by the light irradiation system 100c according to the present embodiment.

In S11, first, the irradiator 11a irradiates the eyes of the subject with light in a predetermined wavelength band (irradiation step).

In S12, the illuminance information acquirer 42 acquires illuminance information (illuminance information acquisition step).

In S13, when the illuminance value is less than the predetermined illuminance value, the subject information outputter 33 outputs information for prompting a change in the state of the subject (information output step).

According to the above control method, the irradiator 11a can irradiate the eyes of the subject with light in a predetermined wavelength band, the illuminance information acquirer 42 can acquire illuminance information, and when the illuminance value is less than the predetermined illuminance value, the subject information outputter 33 can output information prompting a change in the state of the subject. Thus, the light irradiation system 100c can stably irradiate the eyes of the subject with the light in the predetermined wavelength band. Therefore, according to the above control method, for example, irradiation light in a predetermined wavelength band that effectively affects on the mind and body of the subject can be emitted to the eyes of the subject.

### Fifth Embodiment

Another embodiment of the present disclosure will be described below. For the sake of convenience of description, members having the same functions as those of the members described in the above-described embodiments are denoted by the same reference signs, and description thereof is not repeated.

### Configuration of Light Irradiation Device 1d

In the light irradiation system 100c according to the fourth embodiment, the illuminance information acquirer 42 acquires the illuminance information indicating the illuminance value of the light, and when the illuminance value of the light is less than the predetermined illuminance value, the light irradiation system 100c outputs the information prompting a change in the state of the subject. In the fourth embodiment, the light irradiation device 1c includes the irradiator 11a, the subject information outputter 33, the irradiation controller 34, and the time measurer 12a.

In the present embodiment, a light irradiation device 1d may be an installation-type light irradiation device as the same as or similar to the fourth embodiment, and the subject can receive treatment while sitting on a chair, for example.

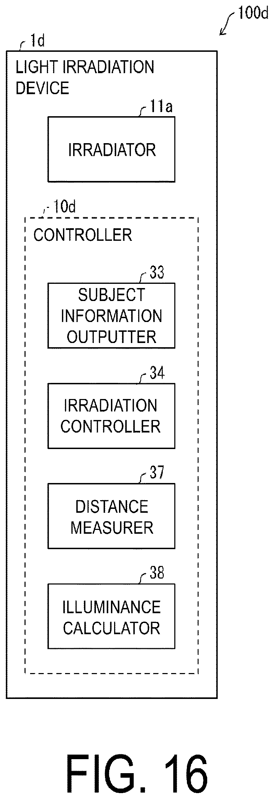

In the present embodiment, the light irradiation device 1d will be described with reference to FIG. 16. FIG. 16 is a block diagram illustrating a configuration example of the light irradiation device 1d according to a fifth embodiment.

As illustrated in FIG. 16, in the present embodiment, the light irradiation device 1d includes an irradiator 11a and a controller 10d, and the controller 10d further includes a distance measurer 37 and an illuminance calculator 38 in addition to a subject information outputter 33 and an irradiation controller 34.

In the present embodiment, the light irradiation device 1d includes the distance measurer 37 and the illuminance calculator 38, so that the illuminance can be calculated without the illuminance information acquirer 42 on the subject side. According to this, only the light irradiation device 1d may be used for treatment, and the illuminance information acquirer 42 need not be provided on the subject side. For example, the subject need not wear glasses or the like provided with the illuminance information acquirer 42.

### Distance Measurer 37

The distance measurer 37 identifies a position of the eye of a subject based on a captured image of the subject, and measures a distance from the irradiator 11a. In other words, the distance measurer 37 measures a distance between the eye of the subject and the irradiator 11a. The captured image of the subject may be acquired by, for example, an infrared camera. The infrared camera may be included in the light irradiation device 1d or may be located separately from the light irradiation device 1d. An image to be captured may be a moving image in order to identify the position of the eye of the subject in real time.

A method by which the distance measurer 37 identifies the position of the eye of the subject is not limited, and a known method may be applied. For example, the distance measurer 37 may identify the position of the eye by detecting the pupil based on a difference between reflected light beams of different angles of lighting. The distance measurer 37 may calculate the distance from the irradiator 11a based on the position of the eye and the size of the eye in the image.

Before starting the treatment, the distance measurer 37 may execute calibration processing of acquiring a captured image of the subject with the subject sitting at a predetermined distance from the irradiator 11a in advance and setting the position of the eye of the subject in the acquired image as a reference position. Thus, the distance can be accurately measured for each subject.

### Illuminance Calculator 38

The illuminance calculator 38 calculates the illuminance value of light at or near the eye of the subject based on the distance measured by the distance measurer 37 and intensity of light emitted from the irradiator 11a. The illuminance calculator 38 may hold information indicating a correspondence relationship of illuminance value to be calculated from the distance and the intensity of the light emitted from the irradiator 11a, that is, a relational equation in advance, and may calculate the illuminance value of the light from the distance and the intensity of the light based on this information.

### Control Method Executed by Light Irradiation Device 1d

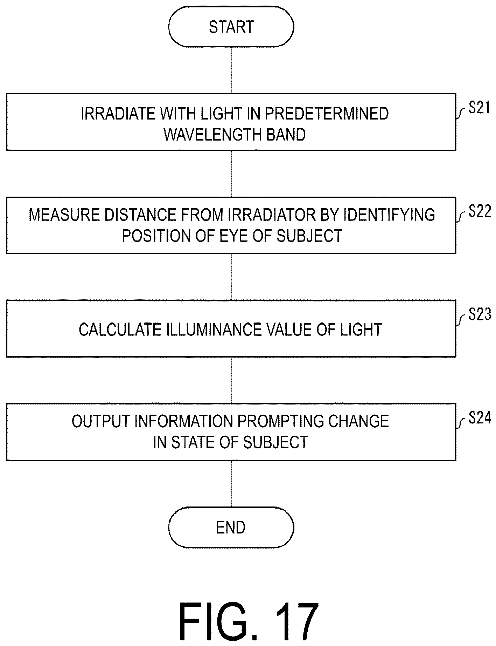

FIG. 17 is a flowchart illustrating a flow of a control method to be executed by the light irradiation device 1d according to the present embodiment.

In S21, first, the irradiator 11a irradiates the eye of the subject with light in a predetermined wavelength band (irradiation step).

In S22, the distance measurer 37 measures a distance from the irradiator 11a by identifying a position of the eye of the subject based on the captured image of the subject (distance measurement step).

In S23, the illuminance calculator 38 calculates an illuminance value of the irradiation light at or near the eye of the subject based on the distance and the intensity of the irradiation light emitted from the irradiator 11a (illuminance calculation step).

In S24, when the illuminance value of the irradiation light at or near the eye of the subject is less than the predetermined illuminance value, the subject information outputter 33 outputs information prompting a change in the state of the subject (subject information output step).

According to the above control method, the irradiator 11a can irradiate the eye of the subject with light in a predetermined wavelength band, the distance measurer 37 can measure the distance from the irradiator 11a, the illuminance calculator 38 can calculate the illuminance of the irradiation light, and the subject information outputter 33 can output information prompting a change in the state of the subject. Thus, the light irradiation system 100d can stably irradiate the eyes of the subject with the light in the predetermined wavelength band. Therefore, according to the above control method, for example, irradiation light in a predetermined wavelength band that effectively affects on the mind and body of the subject can be emitted to the eyes of the subject.

### Variation

In the fifth embodiment, the distance measurer 37 identifies the position of the eye of the subject based on the captured image of the subject. The controller 10d may, for example, identify the subject based on the face or the eyes of the subject in the captured image of the subject. For example, a subject database may store in advance a table that associates the face images of subjects with subject IDs. When a face image of a subject about to receive the treatment matches the image in the subject database, the controller 10d may assign a corresponding ID in the subject database to the subject about to be treated. This eliminates the need for subjects or administrators (medical personnel) to enter subject IDs.

The subject database may store treatment histories such as past irradiation time, date, illuminance, and cumulative illuminance for each subject. The controller 10d may manage treatment for each subject based on these treatment histories in the subject database. For example, when a subject about to be treated sits in front of the light irradiation device 1d, the controller 10d may identify a subject ID corresponding to the subject, as described above, set an irradiation time, illuminance, and the like based on treatment histories corresponding to the subject ID, and start treatment. According to this, appropriate light can be emitted for each subject, and a pain alleviation effect can be given to the subject.

### Conclusion

According to an aspect 1B of the present invention, a light irradiation system includes an irradiator configured to irradiate the eye of a subject with light in a predetermined wavelength band, an illuminance information acquirer positioned facing the irradiator and configured to acquire illuminance information indicating an illuminance value of the light, and a subject information outputter configured to output information prompting a change in a state of the subject when the illuminance value of the light is less than a predetermined illuminance value.

In the light irradiation system according to an aspect 2B of the present invention, in the aspect 1B, the information prompting the change in the state of the subject may be information configured to direct the face of the subject toward the irradiator or information configured to bring the face of the subject closer to the irradiator.

According to an aspect 3B of the present invention, in the aspect 1B or 2B, the light irradiation system may include an irradiation controller configured to adjust intensity of the light emitted from the irradiator based on the illuminance information.

In the light irradiation system according to an aspect 4B of the present invention, in the aspect 3B, the irradiator may include multiple light emitting elements configured to emit light, the multiple light emitting elements may be arranged, and the irradiation controller may cause at least one of the multiple light emitting elements to emit light.

In the light irradiation system according to an aspect 5B of the present invention, in the aspect 4B, the irradiation controller may adjust the intensity of the light emitted from the irradiator by controlling the number of light emitting elements, each of the light emitting elements being caused to emit light among the multiple light emitting elements arranged.

In the light irradiation system according to an aspect 6B of the present invention, in the aspect 5B, the irradiation controller may display an object composed of an element emitting light and an element not emitting light on the irradiator by controlling arrangement of the light emitting elements, each of the light emitting elements being caused to emit light among the multiple light emitting elements arranged.

According to an aspect 7B of the present invention, in any one of the aspects 1B to 6B, the light irradiation system may further include a shielding mechanism configured to reduce illuminance of light at least in a wavelength band different from the predetermined wavelength band that is included in light different from the light.

According to an aspect 8B of the present invention, in any one of the aspects 1B to 7B, the light irradiation system may further include a time measurer configured to measure a time during which the illuminance value of the light is equal to or greater than the predetermined illuminance value as an irradiation time based on the illuminance information, and irradiation by the irradiator may be ended when a length of the irradiation time reaches a predetermined time.

In the light irradiation system according to an aspect 9B of the present invention, in the aspect 8B, the predetermined wavelength band may be from 450 nm to 600 nm.

In the light irradiation system according to an aspect 10B of the present invention, in the aspect 8B or 9B, a length of the predetermined time may be 0.5 hours or more and less than 8 hours.

According to an aspect 11B of the present invention, a light irradiation device includes an irradiator configured to irradiate the eye of a subject with light in a predetermined wavelength band, and a subject information outputter configured to output information prompting a change in a state of the subject when an illuminance value of the light at or near the eye of the subject is less than a predetermined illuminance value.

According to an aspect 12B of the present invention, a light irradiation device includes an irradiator configured to irradiate the eye of a subject with light in a predetermined wavelength band, a distance measurer configured to measure a distance from the irradiator by identifying a position of the eye of the subject based on a captured image of the subject, an illuminance calculator configured to calculate an illuminance value of the light at or near the eye of the subject based on the distance and intensity of the light emitted from the irradiator, and a subject information outputter configured to output information prompting a change in a state of the subject when the illuminance value calculated is equal to or less than a predetermined illuminance value.

According to an aspect 13B of the present invention, a method of controlling a light irradiation system includes irradiating the eye of a subject with light in a predetermined wavelength band, acquiring illuminance information indicating an illuminance value of the light at or near the eye of the subject, and outputting information prompting a change in a state of the subject when the illuminance value of the light at or near the eye is less than a predetermined illuminance value.

According to an aspect 14B of the present invention, a method of controlling a light irradiation system includes irradiating the eye of a subject with light in a predetermined wavelength band from an irradiator, measuring a distance from the irradiator by identifying a position of the eye of the subject based on a captured image of the subject, calculating an illuminance value of the light at or near the eye of the subject based on the distance and intensity of the light emitted from the irradiator, and outputting information prompting a change in a state of the subject when the illuminance value of the light at or near the eye of the subject is less than a predetermined illuminance value.

### Example of Software Implementation

The control blocks (controller 10, 10a, 10b, 10c, and 10d) of the light irradiation systems 100, 100a, 100b, 100c, and 100d may be implemented by logic circuits (hardware) formed in integrated circuits (IC chips) or the like, or may be implemented by software.

In the latter case, the light irradiation systems 100, 100a, 100b, 100c, and 100d include computers that execute the instructions of program, which are software for implementing the functions. The computer includes, for example, one or more processors and a computer-readable recording medium that stores the above program. In the computer, the processor reads the above program from the recording medium and executes the read program to achieve the object of the present disclosure. As the processor, a central processing unit (CPU) can be used, for example. As the recording medium, a "non-transitory tangible medium" such as, for example, a read only memory (ROM), a tape, a disk, a card, a semiconductor memory, a programmable logic circuit, and the like can be used. A random access memory (RAM) for loading the above program may be further provided. The above program may be supplied to the computer via any transmission medium (communication network, broadcast waves, and the like) that can transmit the program. An aspect of the present disclosure may be implemented in the form of data signals embedded in a carrier wave in which the above program is embodied by electronic transmission.

### EXAMPLES

A light irradiation test performed on rats (subjects) will be described below.

### Test Method

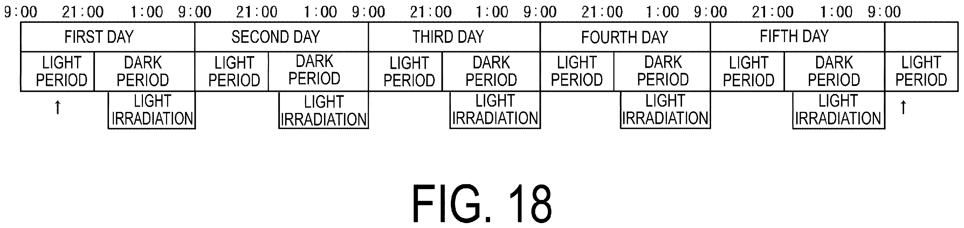

A light irradiation test was conducted on rats (n = 5) to study an irradiation time. FIG. 18 is a diagram illustrating a 5-day irradiation schedule for the light irradiation test conducted on the rats. The schedule in FIG. 18 is a schedule specifically illustrating periods when lighting in a breeding room for rats is ON (light period) and OFF (dark period), a timing of light irradiation to the rats, a length of an irradiation time, and a timing of an analgesic effect test and blood sampling. The test period was five days, with the light period from 9:00 to 21:00 and the dark period from 21:00 to 9:00 the next day.

First, an analgesic test and blood sampling were conducted on the rats at a certain time during the light period on the first day. Thereafter, at a predetermined time during the dark period, the rats were irradiated with light (wavelength: 527 nm, output intensity: 100 Lux) for a predetermined time period. Light irradiation was performed for five consecutive days under the same conditions, and an analgesic test and blood sampling were conducted on the next day of the fifth day during the light period as the same as or similar to the first day.

### Analgesic Test

Using a Randall Selitto analgesic effect measuring device (manufactured by UNICOM Co., Ltd., model number K-201), a pressure stimulus was applied to a tip of the paw of the rat, and a pressure value (mmHg) at which the rat showed an escape response was measured and defined as an avoidance response threshold pressure.

### Analysis of Blood Enkephalin Concentration

Blood enkephalin concentrations (pg/mL) of blood taken from the rats were analyzed. Analysis was conducted using the Rat Enkephalin ELISA Kit (LSBio).

### Statistical Analysis

Results of the analgesic test and results of blood enkephalin concentration analysis are shown as mean ± standard error. The Student's t-test was conducted, and the significance level was set at p < 0.05 and p < 0.01.

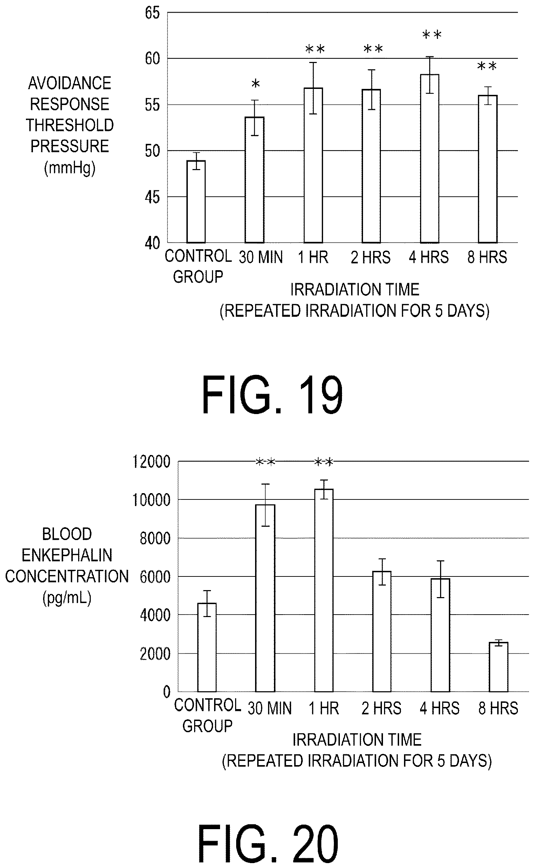

FIG. 19 is a graph showing the results of the analgesic test. In the graph in FIG. 19, the horizontal axis represents the continuous irradiation time when light was repeatedly emitted for five days, and the vertical axis represents the avoidance response threshold pressure (mmHg) of the rats. The greater the avoidance response threshold pressure, the more insensitive the rat is to pain. In the graph in FIG. 11, a group with p < 0.05 is marked with one asterisk (*) and groups with p < 0.01 are marked with two asterisks. The graph in FIG. 19 shows that a rat group irradiated with light for 30 minutes had a greater avoidance response threshold pressure than a control group irradiated with light for less than 30 minutes. In a rat group irradiated with light for one hour, the avoidance response threshold pressure was further greater, but the avoidance response threshold pressures of rat groups irradiated with light for two hours, four hours, and eight hours were almost the same as the avoidance response threshold pressure of the rat group irradiated with light for one hour. Rather, the avoidance response threshold pressure of the rat group irradiated with light for eight hours was lower than the avoidance response threshold pressure of the rat group irradiated with light for four hours. From the above, it was revealed that the light irradiation for 30 minutes or more had a pain alleviation effect on rats. It was also revealed that even when the light irradiation was less than eight hours, the pain alleviation effect on rats was sufficient.

FIG. 20 is a graph showing the results of the blood enkephalin concentration analysis. In the graph in FIG. 20, the horizontal axis represents the continuous irradiation time when light was repeatedly emitted for five days, and the vertical axis represents the blood enkephalin concentration (pg/mL) of the rats. In the graph in FIG. 20, a group with p < 0.05 is marked with one asterisk (*) and groups with p < 0.01 are marked with two asterisks. The graph in FIG. 20 shows that the rat group irradiated with light for 30 minutes had a greater blood enkephalin concentration than the control group irradiated with light for less than 30 minutes. The blood enkephalin concentration of the rat group irradiated with light for 1 hour was greater than that of the rat group irradiated with light for 30 minutes. However, in the rat groups irradiated with light for two hours or more, the blood enkephalin concentration tended to decrease. From the above, it was revealed that the light irradiation for 30 minutes or more had a sufficient pain alleviation effect on rats.

In this example, a pain alleviation effect of light irradiation by the light irradiation system of the invention according to the present disclosure on rats was revealed. It is common not only in rats but also in all animals that an increase blood enkephalin concentration exerts a pain alleviation effect. Therefore, the light irradiation system of the invention according to the present disclosure is applicable to all animals, and is expected to exhibit the pain alleviation effect in all animals, as the same as and/or similar to rats.

In the present disclosure, the invention has been described above based on the various drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, a person skilled in the art can easily make various variations or modifications based on the present disclosure. Note that these variations or modifications are included within the scope of the present disclosure.

### REFERENCE SIGNS

1, 1a, 1b, 1c, 1d Light irradiation device
10, 10a, 10b, 10c, 10d Controller
11, 11a Irradiator
12, 12a Time measurer
13 Presentator
14 Distance measurer
16 Illuminance sensor
18 Communicator
20 Subject information manager
21 Identification information
22 Treatment history information
23 Treatment schedule information
42 Illuminance information acquirer
33 Subject information outputter
34 Irradiation controller
17, 17a Shielding mechanism
37 Distance measurer
38 Illuminance calculator
100, 100a, 100b, 100c, 100d Light irradiation system

## Claims

1. A light irradiation system comprising:
an irradiator configured to irradiate a predetermined part of the body of a subject with light in a predetermined wavelength band;
a time measurer configured to measure an irradiation time of the light in one treatment; and
a controller configured to control the irradiator, wherein
the controller controls the irradiation time to be less than eight hours.

2. The light irradiation system according to claim 1, wherein
the predetermined part of the body of the subject is an eye and/or an ear.

3. The light irradiation system according to claim 1 or 2, wherein
a length of time of the treatment is 0.5 hours or more.

4. The light irradiation system according to claim 1 or 2, wherein
a length of time of the treatment is one hour or less.

5. The light irradiation system according to claim 1 or 2, further comprising:
a subject information manager configured to manage identification information of the subject and treatment history information comprising the irradiation time of the treatment and the number of times of the treatment received by the subject, in association with each other subject by subject; and
a presentator configured to present the treatment history information to the subject corresponding to the treatment history information.

6. The light irradiation system according to claim 5, wherein
the presentator presents information to each subject, the information representing a scheduled end time of the treatment currently being received by the subject.

7. The light irradiation system according to claim 5, further comprising:
a communicator configured to communicate with a communication device of the subject, wherein
the subject information manager manages the identification information about the subject, the treatment history information comprising the irradiation time and the number of times of the treatment received by the subject, and treatment schedule information about a schedule of the treatment scheduled to be received by the subject, in association with each other, and
the treatment schedule information is transmitted to the communication device of the subject corresponding to the treatment schedule information.

8. The light irradiation system according to claim 5, further comprising:
a distance measurer configured to measure a distance between the irradiator and the eye of the subject, wherein
the presentator presents a presentation screen configured to instruct the subject to move a position of the predetermined part of the body according to the distance.

9. The light irradiation system according to claim 8, further comprising:
an illuminance sensor configured to be attached to the subject, wherein
the distance measurer measures the distance between the irradiator and the eye of the subject based on an illuminance value detected by the illuminance sensor.

10. The light irradiation system according to claim 8, further comprising:
an illuminance sensor configured to be attached to the subject, wherein
the controller adjusts an output of light by the irradiator based on an illuminance value detected by the illuminance sensor.

11. The light irradiation system according to claim 1 or 2, further comprising:
a shielding mechanism configured to reduce illuminance of light at least in a wavelength band different from the predetermined wavelength band, the light being comprised in light different from the light in the predetermined wavelength band.

12. The light irradiation system according to claim 2, further comprising:
an illuminance information acquirer positioned facing the irradiator and configured to acquire illuminance information indicating an illuminance value of the light; and
a subject information outputter configured to output information prompting a change in a state of the subject when the illuminance value of the light is less than a predetermined illuminance value.

13. The light irradiation system according to claim 12, wherein
the information prompting the change in the state of the subject is information configured to direct the face of the subject toward the irradiator or information configured to bring the face of the subject closer to the irradiator.

14. The light irradiation system according to claim 12, further comprising:
an irradiation controller configured to adjust intensity of the light emitted from the irradiator based on the illuminance information.

15. The light irradiation system according to claim 14, wherein
the irradiator comprises multiple light emitting elements configured to emit light,
the multiple light emitting elements are arranged, and
the irradiation controller causes at least one of the multiple light emitting elements to emit light.

16. The light irradiation system according to claim 15, wherein the irradiation controller adjusts the intensity of the light emitted from the irradiator by controlling the number of light emitting elements, each of the light emitting elements being caused to emit light among the multiple light emitting elements arranged.

17. The light irradiation system according to claim 16, wherein the irradiation controller displays an object composed of an element emitting light and an element not emitting light on the irradiator by controlling arrangement of the light emitting element being caused to emit light among the multiple light emitting elements arranged.

18. The light irradiation system according to claim 12, wherein
the irradiation time is a time during which the illuminance value of the light is equal to or greater than the predetermined illuminance value, and
the time measurer ends irradiation by the irradiator when a length of the irradiation time reaches a predetermined time.

19. The light irradiation system according to claim 1, wherein
the predetermined wavelength band is from 450 nm to 600 nm.
